# EUROPEAN PATENT APPLICATION

(11) **EP 3 789 401 A1**
(43) Date of publication of application: **10.03.2021**
(21) Application number: 19306066.2
(22) Date of filing: 03.09.2019
(51) Int. Cl.: C07K 16/28, A61K 47/68, A61K 31/5517, A61P 35/00

(54) **AMHRII-BINDING ANTIBODY DRUG CONJUGATES AND THEIR USE THEREOF IN THE TREATMENT OF CANCERS**

(71) Applicant: Gamamabs Pharma, 31106 Toulouse (FR)
(72) Inventor: Prost, Jean-François, 78000 Versailles (FR); Barret, Jean-Marc, 81100 Castres (FR); Dubreuil, Olivier, 31190 Mauressac (FR); D'Hooge, François, 85276 Pfaffenhofen an der Ilm (DE); Lahmar, Mehdi, 55122 Mainz (DE)
(74) Representative: Nony

(57) **Abstract**

The present invention relates to an antibody drug conjugate (ADC) of general formula (I) (D-Lk₁-C(O)-Lk₂-C₂H₄-NH-Lk₃)ₙ-Ab. A pharmaceutical composition comprising, in a pharmaceutically acceptable medium, the said ADC is also concerned by the present invention, as well as the use of this ADC or composition as a medicament, and in particular in the prevention and/or the treatment of an anti-Müllerian hormone type II receptor (AMHRII) expressing cancer in an individual.

SEQUENCE LISTING

<110> GAMAMABS PHARMA

<120> AMHRII-binding antibody drug conjugates and their use thereof in the
treatment of cancers

<130> PR83921

<160> 12

<170> BiSSAP 1.3.6

<210> 1
<211> 13
<212> PRT
<213> Artificial Sequence

<220>
<223> H-CDR1

<400> 1
Lys Ala Ser Gly Tyr Thr Phe Thr Ser Tyr His Ile His
1               5                   10

<210> 2
<211> 17
<212> PRT
<213> Artificial Sequence

<220>
<223> H-CDR2

<400> 2
Trp Ile Tyr Pro Gly Asp Asp Ser Thr Lys Tyr Ser Gln Lys Phe Gln
1               5                   10                  15
Gly

<210> 3
<211> 6
<212> PRT
<213> Artificial Sequence

<220>
<223> H-CDR3

<400> 3
Gly Asp Arg Phe Ala Tyr
1               5

<210> 4
<211> 10
<212> PRT
<213> Artificial Sequence

<220>
<223> L-CDR1

<400> 4
Arg Ala Ser Ser Ser Val Arg Tyr Ile Ala
1               5                   10

<210> 5
<211> 7
<212> PRT
<213> Artificial Sequence

<220>
<223> L-CDR2

<400> 5
Pro Thr Ser Ser Leu Glu Ser
1               5

<210> 6
<211> 10
<212> PRT
<213> Artificial Sequence

<220>
<223> L-CDR3

<400> 6
Leu Gln Trp Ser Ser Tyr Pro Trp Thr Phe
1               5                   10

<210> 7
<211> 115
<212> PRT
<213> Artificial Sequence

<220>
<223> heavy chain variable region

<400> 7
Gln Val Arg Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
His Ile His Trp Val Arg Gln Ala Pro Gly Gln Arg Leu Glu Trp Met
        35                  40                  45
Gly Trp Ile Tyr Pro Gly Asp Asp Ser Thr Lys Tyr Ser Gln Lys Phe
    50                  55                  60
Gln Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Gly Asp Arg Phe Ala Tyr Trp Gly Gln Gly Thr Leu Val Thr
            100                 105                 110
Val Ser Ser
        115

<210> 8
<211> 106
<212> PRT
<213> Artificial Sequence

<220>
<223> light chain variable region

<400> 8
Asp Ile Gln Met Thr Gln Ser Pro Ser Thr Leu Ser Ala Ser Val Gly
1               5                   10                  15
Asp Arg Val Thr Ile Thr Cys Arg Ala Ser Ser Ser Val Arg Tyr Ile
            20                  25                  30
Ala Trp Tyr Gln Gln Lys Pro Gly Lys Ala Pro Lys Leu Leu Thr Tyr
        35                  40                  45
Pro Thr Ser Ser Leu Glu Ser Gly Val Pro Ser Arg Phe Ser Gly Ser
    50                  55                  60
Gly Ser Gly Thr Glu Phe Thr Leu Thr Ile Ser Ser Leu Gln Pro Asp
65                  70                  75                  80
Asp Phe Ala Thr Tyr Tyr Cys Leu Gln Trp Ser Ser Tyr Pro Trp Thr
                85                  90                  95
Phe Gly Gly Gly Thr Lys Val Glu Ile Lys
            100                 105

<210> 9
<211> 446
<212> PRT
<213> Artificial Sequence

<220>
<223> heavy chain

<400> 9
Gln Val Arg Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
His Ile His Trp Val Arg Gln Ala Pro Gly Gln Arg Leu Glu Trp Met
        35                  40                  45
Gly Trp Ile Tyr Pro Gly Asp Asp Ser Thr Lys Tyr Ser Gln Lys Phe
    50                  55                  60
Gln Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Gly Asp Arg Phe Ala Tyr Trp Gly Gln Gly Thr Leu Val Thr
            100                 105                 110
Val Ser Ser Ala Ser Thr Lys Gly Pro Ser Val Phe Pro Leu Ala Pro
        115                 120                 125
Ser Ser Lys Ser Thr Ser Gly Gly Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Gln Thr Tyr Ile Cys Asn Val Asn His Lys Pro Ser Asn Thr Lys
        195                 200                 205
Val Asp Lys Arg Val Glu Pro Lys Ser Cys Asp Lys Thr His Thr Cys
    210                 215                 220
Pro Pro Cys Pro Ala Pro Glu Leu Leu Gly Gly Pro Ser Cys Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210> 10
<211> 213
<212> PRT
<213> Artificial Sequence

<220>
<223> light chain

<400> 10
Asp Ile Gln Met Thr Gln Ser Pro Ser Thr Leu Ser Ala Ser Val Gly
1               5                   10                  15
Asp Arg Val Thr Ile Thr Cys Arg Ala Ser Ser Ser Val Arg Tyr Ile
            20                  25                  30
Ala Trp Tyr Gln Gln Lys Pro Gly Lys Ala Pro Lys Leu Leu Thr Tyr
        35                  40                  45
Pro Thr Ser Ser Leu Glu Ser Gly Val Pro Ser Arg Phe Ser Gly Ser
    50                  55                  60
Gly Ser Gly Thr Glu Phe Thr Leu Thr Ile Ser Ser Leu Gln Pro Asp
65                  70                  75                  80
Asp Phe Ala Thr Tyr Tyr Cys Leu Gln Trp Ser Ser Tyr Pro Trp Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

<210> 11

<211> 556
<212> PRT
<213> Artificial Sequence

<220>
<223> human AMHRII sequence lacking its signal peptide

<400> 11
Pro Pro Asn Arg Arg Thr Cys Val Phe Phe Glu Ala Pro Gly Val Arg
1               5                   10                  15
Gly Ser Thr Lys Thr Leu Gly Glu Leu Leu Asp Thr Gly Thr Glu Leu
            20                  25                  30
Pro Arg Ala Ile Arg Cys Leu Tyr Ser Arg Cys Cys Phe Gly Ile Trp
        35                  40                  45
Asn Leu Thr Gln Asp Arg Ala Gln Val Glu Met Gln Gly Cys Arg Asp
    50                  55                  60
Ser Asp Glu Pro Gly Cys Glu Ser Leu His Cys Asp Pro Ser Pro Arg
65                  70                  75                  80
Ala His Pro Ser Pro Gly Ser Thr Leu Phe Thr Cys Ser Cys Gly Thr
                85                  90                  95
Asp Phe Cys Asn Ala Asn Tyr Ser His Leu Pro Pro Pro Gly Ser Pro
            100                 105                 110
Gly Thr Pro Gly Ser Gln Gly Pro Gln Ala Ala Pro Gly Glu Ser Ile
        115                 120                 125
Trp Met Ala Leu Val Leu Leu Gly Leu Phe Leu Leu Leu Leu Leu Leu
    130                 135                 140
Leu Gly Ser Ile Ile Leu Ala Leu Leu Gln Arg Lys Asn Tyr Arg Val
145                 150                 155                 160
Arg Gly Glu Pro Val Pro Glu Pro Arg Pro Asp Ser Gly Arg Asp Trp
                165                 170                 175
Ser Val Glu Leu Gln Glu Leu Pro Glu Leu Cys Phe Ser Gln Val Ile
            180                 185                 190
Arg Glu Gly Gly His Ala Val Val Trp Ala Gly Gln Leu Gln Gly Lys
        195                 200                 205
Leu Val Ala Ile Lys Ala Phe Pro Pro Arg Ser Val Ala Gln Phe Gln
    210                 215                 220
Ala Glu Arg Ala Leu Tyr Glu Leu Pro Gly Leu Gln His Asp His Ile
225                 230                 235                 240
Val Arg Phe Ile Thr Ala Ser Arg Gly Gly Pro Gly Arg Leu Leu Ser
                245                 250                 255
Gly Pro Leu Leu Val Leu Glu Leu His Pro Lys Gly Ser Leu Cys His
            260                 265                 270
Tyr Leu Thr Gln Tyr Thr Ser Asp Trp Gly Ser Ser Leu Arg Met Ala
        275                 280                 285
Leu Ser Leu Ala Gln Gly Leu Ala Phe Leu His Glu Glu Arg Trp Gln
    290                 295                 300
Asn Gly Gln Tyr Lys Pro Gly Ile Ala His Arg Asp Leu Ser Ser Gln
305                 310                 315                 320
Asn Val Leu Ile Arg Glu Asp Gly Ser Cys Ala Ile Gly Asp Leu Gly
                325                 330                 335
Leu Ala Leu Val Leu Pro Gly Leu Thr Gln Pro Pro Ala Trp Thr Pro
            340                 345                 350
Thr Gln Pro Gln Gly Pro Ala Ala Ile Met Glu Ala Gly Thr Gln Arg
        355                 360                 365
Tyr Met Ala Pro Glu Leu Leu Asp Lys Thr Leu Asp Leu Gln Asp Trp
    370                 375                 380
Gly Met Ala Leu Arg Arg Ala Asp Ile Tyr Ser Leu Ala Leu Leu Leu
385                 390                 395                 400
Trp Glu Ile Leu Ser Arg Cys Pro Asp Leu Arg Pro Asp Ser Ser Pro
                405                 410                 415
Pro Pro Phe Gln Leu Ala Tyr Glu Ala Glu Leu Gly Asn Thr Pro Thr
            420                 425                 430

Ser Asp Glu Leu Trp Ala Leu Ala Val Gln Glu Arg Arg Arg Pro Tyr
        435                 440                 445
Ile Pro Ser Thr Trp Arg Cys Phe Ala Thr Asp Pro Asp Gly Leu Arg
    450                 455                 460
Glu Leu Leu Glu Asp Cys Trp Asp Ala Asp Pro Glu Ala Arg Leu Thr
465                 470                 475                 480
Ala Glu Cys Val Gln Gln Arg Leu Ala Ala Leu Ala His Pro Gln Glu
                485                 490                 495
Ser His Pro Phe Pro Glu Ser Cys Pro Arg Gly Cys Pro Pro Leu Cys
            500                 505                 510
Pro Glu Asp Cys Thr Ser Ile Pro Ala Pro Thr Ile Leu Pro Cys Arg
        515                 520                 525
Pro Gln Arg Ser Ala Cys His Phe Ser Val Gln Gln Gly Pro Cys Ser
    530                 535                 540
Arg Asn Pro Gln Pro Ala Cys Thr Leu Ser Pro Val
545                 550                 555

<210> 12
<211> 17
<212> PRT
<213> Artificial Sequence

<220>
<223> signal peptide of human AMHRII

<400> 12
Met Leu Gly Ser Leu Gly Leu Trp Ala Leu Leu Pro Thr Ala Val Glu
1               5                   10                  15
Ala

## Description

### FIELD OF THE INVENTION

The present invention relates to novel antibody drug conjugates (ADC) binding the human anti-Müllerian hormone type II receptor (AMHRII), in particular for their use in the field of gynecologic and non-gynecologic cancers treatment.

### BACKGROUND OF THE INVENTION

The human anti-Müllerian hormone (AMH) is a glycoprotein of 560 amino acids, a member of the TGF-β family. It is a hormone secreted by the Sertoli cells of the fetal testis, which causes degeneration of the Müllerian duct. It is expressed in the adult in the Sertoli cells and Leydig cells (testis) and the granulosa cells (ovary). It plays a role in the activity of the adult ovary in the regulation of folliculogenesis.

The anti-Müllerian hormone type II receptor (AMHRII) is a peptide of 573 amino acids and possesses serine-threonine kinase activity. It is involved in the regression of the Müllerian duct associated with the development of the human reproductive system. The Müllerian duct atrophies in men, where it only forms the prostatic vesicle and the sessile hydatid, but persists in women, where it gives rise to the Fallopian tubes, uterus and the greater part of the vagina. This receptor is frequently expressed on human ovarian epithelial tumor cells.

More particularly, it has been shown that AMH inhibits proliferation and induces apoptosis of AMH type II receptor (AMHRII) -positive tumor cells, especially progenitor cancer cells. Various reports have moreover described the expression of AMHRII in human gynecologic cancers including ovarian tumors.

It has moreover recently been described the detection of AMHRII mRNA in several primary cancer tissues including melanoma, lung, head and neck, colorectal and breast cancers. AMHRII protein expression was later confirmed by ImmunoHistoChemistry (IHC). Using this same method, over 1000 frozen samples covering 16 different cancer types were tested, which demonstrated AMHRII expression in over 50% of hepatocarcinoma, colorectal, lung and renal cancer samples. Preliminary FACS analysis of fresh ovarian and colorectal tumor samples have demonstrated comparable expression levels with mean values of 50,000 and 40,000 AMHRII receptors per cell, respectively. It has thus been shown that AMHRII is expressed in many different histological types of gynecological and non-gynecological solid tumors.

International application WO 2008/053330 describes a murine 12G4 monoclonal antibody directed against AMHRII for treating ovarian cancers.

International application WO 2011/141653 describes mutated humanized 12G4 antibodies, or fragments thereof, possessing an affinity at least equal to that of the corresponding unmutated chimeric antibody, specificity with respect to the AMHRII receptor, and not eliciting an immune reaction.

International application WO 2017/025458 moreover describes an antibody drug conjugate, named GM103, that binds to AMHRII and can be used to treat AMHRII expressing cancers. GM103 was obtained by conjugation of the humanized low fucose 3C23K antibody with a MMAE drug payload. 3C23K is indicated as being obtained as described in WO 2011/141653.

However, there still remains a need in the art for further tools for the therapy of cancers, that may be alternative or complementary to the existing therapies. In particular, it remains a need for further tools having improved properties in the therapy of cancers compared to the already existing therapy, and in particular to the already existing antibody drug conjugates.

### SUMMARY OF THE INVENTION

According to a first object, the present invention relates to an antibody drug conjugate (ADC) of general formula (I):

(D-Lk₁-C(O)-Lk₂-C₂H₄-NH-Lk₃)ₙ-Ab (I)

wherein:
- D represents a PBD dimer of formula (II): 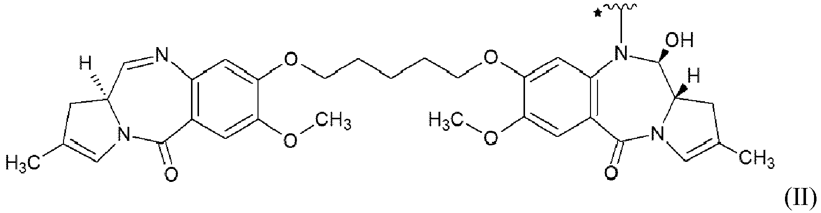 in which ★ represents the link to the Lk₁ group;
- Lk₁ represents a cathepsin B-cleavable valine-alanine dipeptide of formula (III): 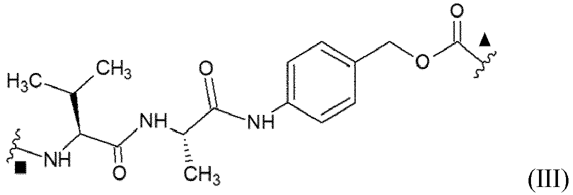 in which ▲ represents the link to the D group and ■ represents the link to the -C(O)-;
- Lk₂ represents a group comprising at least one ethylene glycol unit -CH₂-CH₂-O-;
- Lk₃ represents a linker of formula (IV): 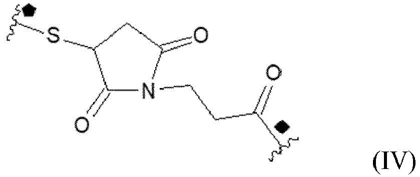 in which ◆ represents the link to the -C₂H₄- group and 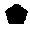 represents the link to the antibody or its fragment thereof;
- n is an integer from 1 to 10; and
- Ab represents an anti-AMHRII antibody, or a fragment thereof, comprising:
   (a) a heavy chain wherein the variable domain comprises:
      - a H-CDR1 having a sequence set forth as SEQ ID NO: 1;
      - a H-CDR2 having a sequence set forth as SEQ ID NO: 2; and
      - a H-CDR3 having a sequence set forth as SEQ ID NO: 3;
      and
   (b) a light chain wherein the variable domain comprises:
      - a L-CDR1 having a sequence set forth as SEQ ID NO: 4,
      - a L-CDR2 having a sequence set forth as SEQ ID NO: 5; and
      - a L-CDR3 having a sequence set forth as SEQ ID NO: 6.

The inventors have unexpectedly shown that while an ADC of the present invention has an *in vitro* binding to AMHRII and an *in vitro* potency lower than the one of an ADC different from the invention (GM103) but also targeting AMHRII, the results obtained *in vivo* with an ADC of the invention are superior to the said ADC outside the invention.

In particular, the inventors unexpectedly demonstrated that an ADC according to the invention is more effective than an ADC outside the invention but also targeting AMHRII. An ADC according to the invention indeed allows obtaining, even when used at a concentration inferior to the one of the comparative ADC, a higher number of tumor free survivors among individual presenting an AMHRII expressing tumor.

Moreover, the inventors unexpectedly demonstrated that an ADC according to the invention is more effective than an ADC outside the invention, but also targeting AMHRII, to reduce *in vivo* the growth of an AMHRII expressing tumor. Furthermore, the inventors unexpectedly demonstrated that an ADC according to the invention is more effective than an ADC outside the invention, but also targeting AMHRII, to significantly increase the number percent of survival among the individuals treated and presenting an AMHRII expressing tumor.

The inventors also unexpectedly demonstrated that an ADC according to the invention possess a significantly higher half-life compared to an ADC outside the invention, but also targeting AMHRII.

Finally, it is surprisingly demonstrated that an ADC according to the invention, while being more effective *in vivo* than an ADC outside the invention, but also targeting AMHRII, is also less toxic than the said different ADC.

In a particular embodiment, LK₂ represents a group consisting of from 2 to 14 ethylene glycol units, in particular of from 4 to 12 ethylene glycol units, more particularly of from 6 to 10 ethylene glycol units, and even more particularly of 8 ethylene glycol units.

In an embodiment of the invention, LK₂ represents a linear group consisting of from 2 to 14 ethylene glycol units, in particular a linear group consisting of from 4 to 12 ethylene glycol units, more particularly a linear group consisting of from 6 to 10 ethylene glycol units, and even more particularly a linear group consisting of 8 ethylene glycol units.

In particular, n can be an integer from 1 to 8, in particular from 1 to 6, more particularly from 1 to 4, even more particularly 1 or 2 and can more particularly be 2.

In a particular embodiment, Ab represents an antibody, or a fragment thereof, wherein the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8.

More particularly, Ab can represent an antibody wherein the heavy chain of said antibody has the amino acid sequence set forth as SEQ ID NO: 9 and/or the light chain of said antibody has the amino acid sequence set forth as SEQ ID NO: 10, and in particular represent an antibody wherein the heavy chain of said antibody has the amino acid sequence set forth as SEQ ID NO: 9 and the light chain of said antibody has the amino acid sequence set forth as SEQ ID NO: 10.

In a particular embodiment, D, Lk1 and Lk3 are as defined above and:
- Lk₂ represents a linear group consisting of 8 ethylene glycol units;
- n is 1 or 2, and in particular 2; and
- Ab represents:
   an antibody, or a fragment thereof, wherein the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8; or
   an antibody wherein the heavy chain of said antibody has the amino acid sequence set forth as SEQ ID NO: 9 and/or the light chain of said antibody has the amino acid sequence set forth as SEQ ID NO: 10, and in particular represent an antibody wherein the heavy chain of said antibody has the amino acid sequence set forth as SEQ ID NO: 9 and the light chain of said antibody has the amino acid sequence set forth as SEQ ID NO: 10.

The inventors indeed generated a novel antibody drug conjugate having unexpected properties as an anti-cancer agent, and in particular having improved anti-tumor properties compared to already available treatments. More particularly, and against all expectations, the inventors obtained a novel ADC which, as demonstrated in the examples of the present text, possess improved properties compared to the above-mentioned ADC named GM103.

A second object of the invention is a pharmaceutical composition comprising, in a pharmaceutically acceptable medium, at least one ADC as defined above.

A further object of the invention is an antibody drug conjugate (ADC) according to the invention for its use as a medicament.

Another object of the invention is a composition according to the invention for its use as a medicament.

Another object of the invention relates to an ADC of the invention, or a composition of the invention, for its use in the prevention and/or the treatment of an anti-Müllerian hormone type II receptor (AMHRII) expressing cancer in an individual.

In a particular embodiment, the cancer is selected from the group consisting of:
- non-gynecologic cancers, in particular selected from the group consisting of colon cancer; liver cancer; hepatocellular carcinoma; testis cancer; thyroid cancer; gastric cancer; gastrointestinal cancer; bladder cancer; lung cancer, in particular non-small cell lung cancer (NSCLC); pancreatic cancer; pancreatic cancer; head and neck cancer; kidney cancer; liposarcoma; fibrosarcoma; pleuramesothelioma; melanoma; sarcoma; brain cancer; osteocarcinoma; breast cancer; prostate cancer; leukemia and hematological cancers; and
- gynecologic cancers, in particular selected from the group consisting of ovarian cancer, in particular metastatic ovarian cancer; serous cancer; hypernephroma; endometrioid cancers; colloidal epithelium cancers; germ cell cancers; endometrial cancer; mixed Müllerian malignant tumor of the uterus; leiomyosarcoma and endometrial stromal sarcoma.

In a further embodiment, the ADC of the invention or the composition of the invention is administered to the said individual intravenously or at the cancer localization, i.e. *in situ.*

In particular, the said individual can be a mammal, and in particular can be a human being.

### DESCRIPTION OF THE FIGURES

**Figure 1** illustrates the results obtained when comparing (i) the binding of the ADC GM103 and an ADC according to the invention (GM104) by Surface Plasmon Resonance and (ii) the anti-proliferation effect of these ADCs on COV434 AMHRII transfected cells (∼80,000 receptors) after 72h of incubation with the said ADCs. Accordingly, the Table of Figure 1 provides:
   - the IC50 values (nM/pM) of GM104 and GM103 in the first line, from left to right;
   - the KD (nM) of GM104 and GM103 to (i) AMHR2 (second line from left to right); (ii) FcRn (third line from left to right); and (iii) FcyR (Fourth line from left to right).
**Figure 2** illustrates the *in vivo* dose dependent efficacy of an ADC according to the invention on COV434 AMHRII transfected cell line tumor xenografted in nude mice.
   GM104, an ADC according to the invention, is administered to mice having tumors once intravenously at 0.1 mg/kg, 0.3 mg/kg and 1.0 mg/kg while a comparative ADC outside the invention, GM103, was administered once intravenously at 5 mg/kg. A control was performed with mice receiving only the vehicle (PBS).
   Tumor volumes of the mice are measured during the 36 days following the day of administration of the ADC or of PBS.
   Ordinate: tumor volume (mm³) mean values for each treatment group (n=8)
   Abscissa: days after start of the treatment.

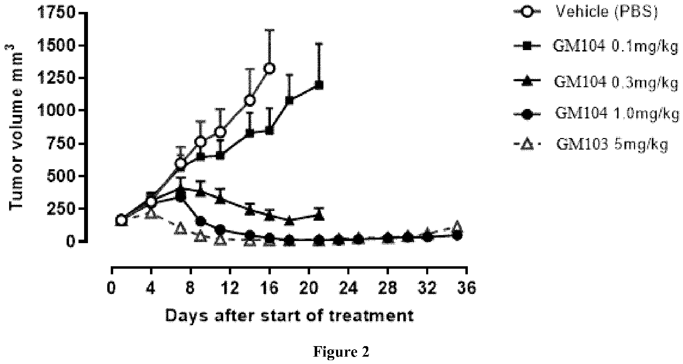

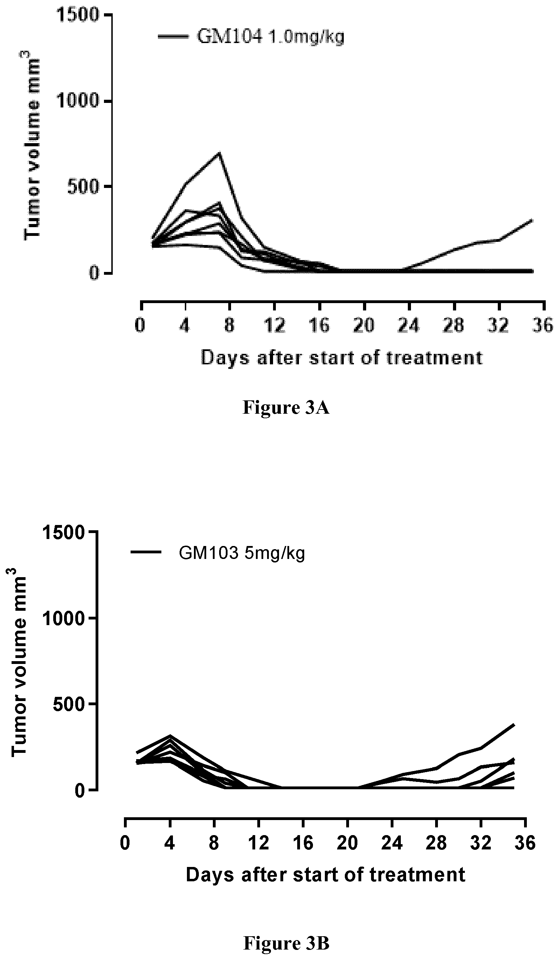

**Figure 3A** illustrates the evolution of the tumor volumes for the 8 members of the group treated with GM104, an ADC according to the invention, once intravenously at 1.0 mg/kg, over the 36 days following said administration in the same experiment as in Figure 2.
   Ordinate: tumor volume (mm³) for each of the 8 mice of the treated group.
   Abscissa: days after start of the treatment.
**Figure 3B** illustrates the evolution of the tumor volumes for the 8 members of the group treated with GM103, an ADC outside the invention, once intravenously at 5.0 mg/kg, over the 36 days following said administration in the same experiment as in Figure 2.
   Ordinate: tumor volume (mm³) for each of the 8 mice of the treated group.
   Abscissa: days after start of the treatment.

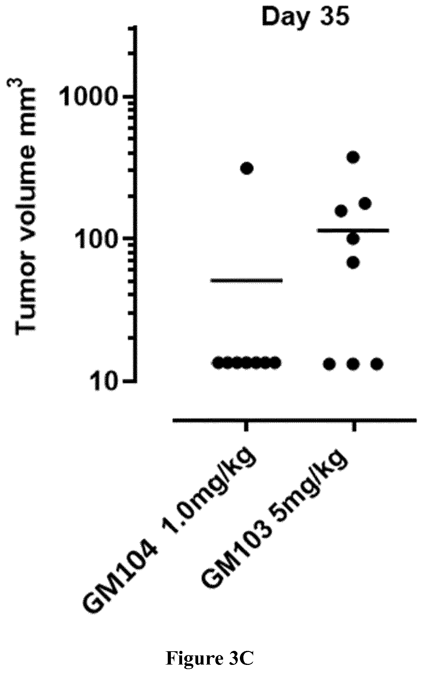

**Figure 3C** illustrates the tumor volumes at day 35 following the administration of an ADC in the same experiment as in Figure 2, for the 8 members of the group treated with GM103 once intravenously at 5.0 mg/kg, and for the 8 members of the group treated with GM104 once intravenously at 1.0 mg/kg.
   The line in each column represents the mean volume of the 8 tumors.
   Ordinate: tumor volume (mm³).
   Abscissa: left column: the 8 members of the group treated with GM104 once intravenously at 1.0 mg/kg; right column: the 8 members of the group treated with GM103 once intravenously at 5.0 mg/kg.

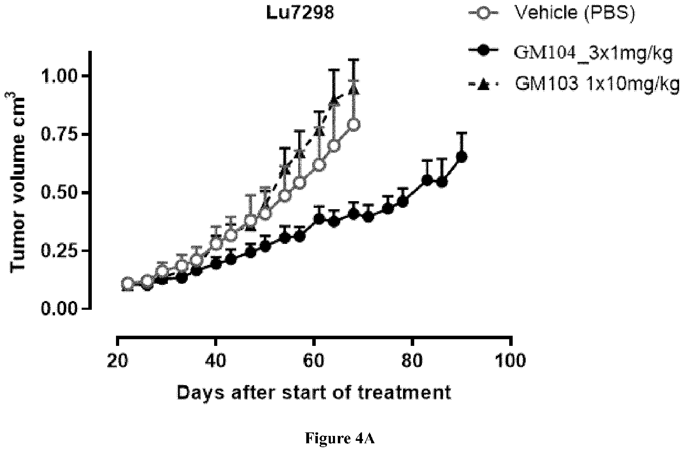

**Figure 4A** illustrates the *in vivo* efficacy of an ADC according to the invention on LU7298, a patient derived xenograft (PDX) from human non-small cell lung cancer patient (NSCLC) implanted in female NMR1 nude mice (NMR1: nu/nu female, Janvier).
   GM104, an ADC according to the invention, is administered to mice having tumors intravenously three times at 1.0 mg/kg (q4dx3) while a comparative ADC outside the invention, GM103, was administered once intravenously at 10 mg/kg. A control was performed with mice receiving only the vehicle (PBS).
   Tumor volumes of the mice are measured two times a week. All mice were individually sacrificed at tumor volume > 1.5 cm³ (used as survival parameter) or at final day 111, and an autopsy was done during the 111 days following the start of the treatments.
   Ordinate: mean tumor volume (cm³) values for each treatment group (n=8)
   Abscissa: days after start of the treatment.

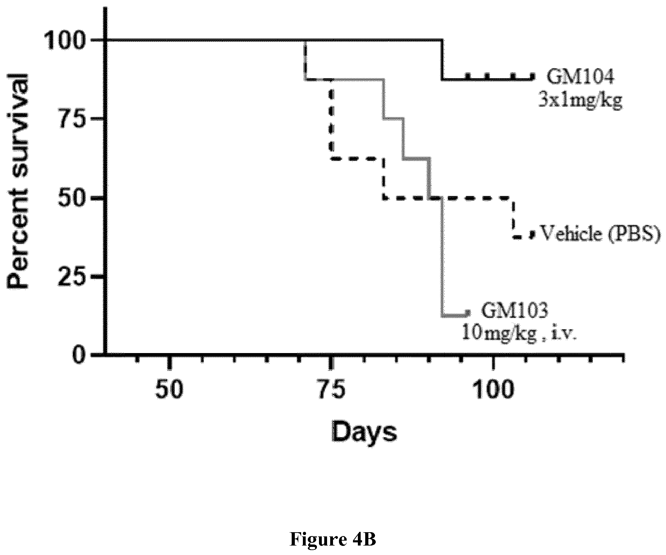

**Figure 4B** illustrates the percent of survival over time of the 3 groups of mice from the experiment of Figure 4A.
   Ordinate: Percent survival.
   Abscissa: days after start of the treatment.

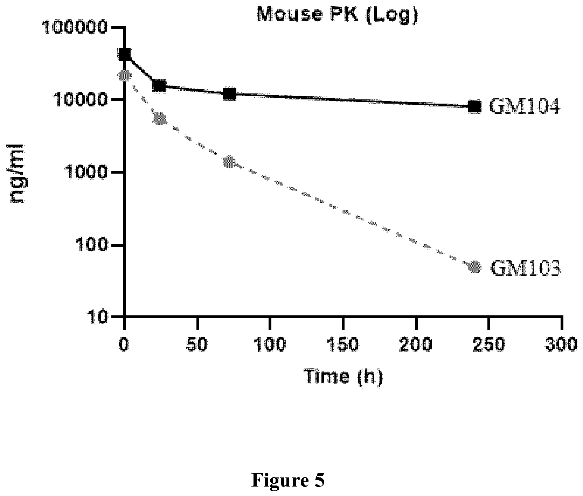

**Figure** 5 illustrates the comparative half-lives of GM103 and GM104 through pharmacokinetics performed in male NMRI nude mice (NMRI: nu/nu male, Janvier); with 6 animals per groups. The animals were split in two sub-groups of 3 mice for monitoring at 5 min and 72 hours and at 24h and 240h. Serum concentration of ADC was monitored using an ELISA method using an antibody targeted at the protein portion of the ADC. All test items were injected intravenously as a single injection at 1.0 mg/kg.
   Pharmacokinetic evaluation of serum data was performed using WinNonlin 8.0 (Pharsight Corporation, Mountain View, CA, USA) and a non-compartmental model was employed.
   Ordinate: ADC concentration (ng/mL) in mouse serum samples;
   Abscissa: time (hours).

### DETAILED DESCRIPTION OF THE INVENTION

### Definitions

The term *"antibody"* used in the present text refers to immunoglobulin molecules and immunologically active portions of immunoglobulin molecules, i.e., molecules that contain:
- an antigen binding site that immunospecifically binds AMHRII; and
- at least one conjugation site cysteine for covalent bonding to Lk₃ through its sulfhydryl group as mentioned in formula (I) of the present invention.

As such, the term antibody encompasses not only whole antibody molecules, but also antibody fragments as well as variants (including derivatives) of antibodies and antibody fragments, having the combined properties mentioned above. In natural antibodies, two heavy chains are linked to each other by disulfide bonds and each heavy chain is linked to a light chain by a disulfide bond. There are two types of light chain, lambda (I) and kappa (k). In the context of this invention, the term *"antibody"* also encompasses chimeric or humanized variants of an antibody.

As used herein, "antibody mutant" or "antibody variant" refers to an amino acid sequence variant of the species-dependent antibody wherein one or more of the amino acid residues of the species-dependent antibody have been modified. Such mutants necessarily have less than 100% sequence identity or similarity with the species-dependent antibody. In one embodiment, the antibody mutant will have an amino acid sequence having at least 75% amino acid sequence identity or similarity with the amino acid sequence of either the heavy or light chain variable domain of the species-dependent antibody, more preferably at least 80%, more preferably at least 85%, more preferably at least 90%, and most preferably at least 95%. Identity or similarity with respect to this sequence is defined herein as the percentage of amino acid residues in the candidate sequence that are identical (i.e same residue) or similar (i.e. amino acid residue from the same group based on common side-chain properties, see below) with the species-dependent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations, κ and λ light chains refer to the two major antibody light chain isotypes.

As used herein the term "complementarity determining region" or "CDR" refers to the part of the two variable chains of antibodies (heavy and light chains) that recognize and bind to the particular antigen. The CDRs are the most variable portion of the variable chains and provide the antibody with its specificity. There are three CDRs on each of the variable heavy (VH) and variable light (VL) chains and thus there are a total of six CDRs per antibody molecule. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VHCDR3 is located in the variable domain of the heavy chain of the antibody in which it is found, whereas a VLCDR1 is the CDR1 from the variable domain of the light chain of the antibody in which it is found. An antibody that binds AMHRII will have a specific VH region and the VL region sequence, and thus specific CDR sequences. Antibodies with different specificities (i.e. different combining sites for different antigens) have different CDRs. Although it is the CDRs that vary from antibody to antibody, only a limited number of amino acid positions within the CDRs are directly involved in antigen binding. These positions within the CDRs are called specificity determining residues (SDRs).

"Framework regions" (hereinafter FR) are those variable domain residues other than the CDR residues. Each variable domain typically has four FRs identified as FRl, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36- 49 (HCFR2), 66-94 (HCFR3), and 103-113 (HCFR4) in the heavy chain residues.

The term *"chimeric antibody"* refers to an engineered antibody which comprises a VH domain and a VL domain of an antibody derived from a non-human animal, a CH domain and a CL domain of another antibody, in particular a human antibody. As the non-human animal, any animal such as mouse, rat, hamster, rabbit or the like can be used.

The term *"humanized antibody"* refers to antibodies in which the framework or "complementarity determining regions" (CDR) have been modified to comprise the CDR from a donor immunoglobulin of different specificity as compared to that of the parent immunoglobulin. In a preferred embodiment, a mouse CDR is grafted into the framework region of a human antibody to prepare the "humanized antibody".

The term *"AMHRII"* relates to the Anti-Müllerian Hormone type II Receptor, and in particular the human Anti-Müllerian Hormone type II Receptor. The sequence of the human AMHRII (lacking the signal peptide MLGSLGLWALLPTAVEA (SEQ ID NO: 12)) is:
PPNRRTCVFFEAPGVRGSTKTLGELLDTGTELPRAIRCLYSRCCFGIWNLTQDRAQVE MQGCRDSDEPGCESLHCDPSPRAHPSPGSTLFTCSCGTDFCNANYSHLPPPGSPGTPG SQGPQAAPGESIWMALVLLGLFLLLLLLLGSIILALLQRKNYRVRGEPVPEPRPDSGR DWSVELQELPELCFSQVIREGGHAVVWAGQLQGKLVAIKAFPPRSVAQFQAERALYE LPGLQHDHIVRFITASRGGPGRLLSGPLLVLELHPKGSLCHYLTQYTSDWGSSLRMAL SLAQGLAFLHEERWQNGQYKPGIAHRDLSSQNVLIREDGSCAIGDLGLALVLPGLTQ PPAWTPTQPQGPAAIMEAGTQRYMAPELLDKTLDLQDWGMALRRADIYSLALLLWE ILSRCPDLRPDSSPPPFQLAYEAELGNTPTSDELWALAVQERRRPYIPSTWRCFATDPD GLRELLEDCWDADPEARLTAECVQQRLAALAHPQESHPFPESCPRGCPPLCPEDCTSI PAPTILPCRPQRSACHFSVQQGPCSRNPQPACTLSPV (SEQ ID NO: 11). Several variants of the human AMHRII are also known. The sequences for these isoforms are provided by the identifiers Q16671-2 and Q16671-3 at the web site uniprot.org.

AMHRII is expressed at the cell membrane of gynecologic and non-gynecologic cancer tissues with a variable frequency depending of the cancer type which is considered. Illustratively, AMHRII is expressed more frequently by cancer cells derived from tumor tissue originating from patients affected with colorectal cancer than by cancer cells derived from tumor tissue originating from patients affected with a head and neck cancer. This means that these two types of cancers are eligible for an anti-cancer treatment targeting AMHRII, but that such an anti-cancer treatment will be less frequently relevant for treating patients affected with a head and neck cancer.

Any cancer mentioned in the present text may be treated by an ADC according to the invention, provided that cancer cells from the said cancer express AMHRII at their membrane, thus provided that the presence of AMHRII proteins at the cancer cell membrane can be detected or determined according to any method well known in the art.

Thus, a ADC according to the invention is effective for treating a plurality of distinct kinds of cancers, provided that the AMHRII target protein is expressed at the cancer cells membrane.

Incidentally, in particular in the field of anti-cancer active ingredients consisting of target-binding molecules, e.g. ADC, the situation wherein the same active ingredient is effective for treating a plurality of distinct cancers is not unprecedented. Illustratively, the anti-PDl antibody named pembrolizumab has been authorized by the US Food and Drug Administration (FDA) as an active ingredient useful in the treatment of a variety of distinct kinds of cancers, provided that the said cancers share the same physiological features.

Thus, an individual affected with a cancer may be treated for the said cancer with an ADC as described herein when AMHRII membrane expression by the cancer cells previously collected from the said individual is detected or otherwise determined by an appropriate method.

In some embodiments, expression of AMHRII at the cell membrane of cancer cells encompasses that the said cancer cells express AMHRII at a given quantifiable level or higher than the said quantifiable level.

Thus, according to some embodiments, responsiveness of an individual affected with a cancer according to the invention to a treatment with an ADC of the invention may be assessed by determining whether cancer cells from a sample previously collected from the said individual express AMHRII at their membrane.

According to some embodiments, responsiveness of an individual affected with a cancer according to the invention to a treatment with an ADC according to the invention may be assessed by determining whether cancer cells from a sample previously collected from the said individual express AMHRII at their membrane above a determined threshold value.

The AMHRII membrane expression level that may be used in some embodiments for determining the responsiveness of a patient affected with a disease according to the invention to a treatment with an ADC of the invention may be assessed with a variety of techniques, which include (i) the percentage of disease cells contained in a cancer sample that express AMHRII at their membrane, (ii) the mean number of AMHRII proteins at the cancer cell membrane and (iii) the FACS AMHRII signal profile of the cancer cells contained in a tested cancer cell sample.

According to some embodiments, cancer cells comprised in a tumor sample previously collected for an individual affected with a cancer according to the invention may be assessed as expressing membranous AMHRII when membranous AMHRII is detected in 5% or more of the cancer cells comprised in the said cancer sample.

Thus, in some embodiments, an individual affected with a cancer according to the invention is determined as being responsive to a treatment with an ADC of the invention when 5% or more of the cancer cells comprised in a cancer sample previously collected from the said individual express AMHRII at their membrane.

Methods for determining the frequency (e.g. the percentage) of cancer cells expressing membrane AMHRII proteins are well known in the art (see for example WO2018189379).

According to some embodiments, responsiveness of a patient affected with a cancer according to the invention to a cancer treatment with an ADC of the invention may be assessed by determining the mean number of AMHRII proteins present at the membrane of the cancer cells contained in a cancer sample previously collected from the said patient.

In some embodiments, a patient affected with a cancer according to the invention may be classified as responsive to a treatment with an ADC of the invention when the mean number of membrane AMHRII proteins expressed by the cancer cells contained in a cancer sample previously collected from the said patient is of 10 000 AMHRII proteins or more.

Assessing the number of AMHRII proteins expressed at the cancer cell membrane may be performed by using conventional methods comprising (a) a step of incubating a sample containing the cells from a cancer tissue sample previously collected from the patient with a detectable compound that binds specifically with AMHRII protein, such as a fluorescently labeled anti-AMHRII antibody, and further (b) a step of determining the number of the said detectable compounds, e.g. the number of fluorescently labeled anti-AMHRII antibodies, bound to each tested cell from the said sample. Assessing the number of AMHRII proteins expressed at the cancer cell membrane may be, for instance, performed by using the well-known Fluorescence Activated Cell Sorting (FACS) technique, as it is shown in the examples herein.

In still other embodiments, a patient affected with a cancer may be classified as responsive to a treatment with an ADC of the invention by analysis of the AMHRII FACS profile of the cancer cells contained in a cancer sample previously collected from the said patient.

According to these still other embodiments, a patient affected with a cancer according to the invention may be classified as responsive to a treatment with an ADC of the invention when, in a method of fluorescence activated cell sorting (FACS), the ratio of (i) the mean fluorescence intensity of the cancer cells incubated with an anti-AMHRII fluorescently labeled antibody to (ii) the mean fluorescence intensity (MFI) value obtained from cancer cells incubated with an isotypic fluorescently labeled antibody is of 1.5 or more.

For determining the said mean fluorescence intensity ratio, both the isotypic antibody and the anti-AMHRII antibody are labeled with the same fluorescent agent, such as the Alexa Fluor 488 dye commercialized by the Company ThermoFisher Scientific., as shown in WO2018189379.

In some further embodiments, responsiveness of a disease individual to a treatment with an ADC of the invention may be determined by calculating an AMHRII expression score allowing to discriminate between (i) membrane AMHRII-expressing cancer cells derived from cancers that may be treated with an AMHRII-binding agent and (ii) membrane AMHRII-expressing cancer cells derived from cancers that may not be treated with an AMHRII-binding agent.

Thus, the inventors have determined that patients affected with a cancer of the present text, who are especially eligible to a cancer treatment with an ADC of the invention encompass those having cancers expressing AMHRII at the cell membrane at a sufficiently high level for consisting in relevant cell targets to be destroyed.

Then, according to these further embodiments, the inventors have determined that a minimal AMHRII expression level measured in a cancer cell sample from a disease patient may confirm that the said patient is responsive to a treatment with an ADC of the invention and that the said patient may thus be treated by an ADC described here-in.

Responsiveness of an individual affected with a cancer to a treatment with an ADC of the invention may thus also be determined when AMHRII expression level by cancer cells comprised in a sample previously collected from the said individual is assessed by both determining (i) the frequency of cancer cells expressing membranous AMHRII, e.g. the percentage of tumor cells expressing AMHRII at their membrane and (ii) the level of AMHRII membrane expression by the said cancer cells, e.g. the mean number of membranous AMHRII proteins per cell.

Thus, in some of these further embodiments, responsiveness of a patient affected with a cancer according to the invention to an ADC of the invention in a sample of cancer cells previously collected from the said patient, may be assessed by determining that (i) the cancer cells contained in the said sample exhibit a minimal mean number of human AMHRII proteins at their membrane and that (ii) the frequency of the cells expressing human AMHRII at their membrane, e.g. the percentage of cells expressing human AMHRII at their membrane, is of at least a threshold value.

Accordingly, it is also described herein a further method that may also be used for determining a specific AMHRII expression score value allowing to discriminate between (i) cancer patients that are not eligible to a cancer treatment with an ADC of the invention, i.e. cancer patients that are not responsive to a cancer treatment with an antibody of the invention, or a cell or a composition of the invention, and (ii) cancer patients that are eligible to a cancer treatment with an antibody of the invention, or a cell or a composition of the invention, i.e. cancer patients that are responsive to a cancer treatment with an antibody of the invention, or a cell or a composition of the invention.

More precisely, according to embodiments of the above method, patients affected with a cancer described herein and who may be treated against cancer with an ADC of the invention may be preferably those for which an AMHRII expression score is of 1.0 or more has been determined, which includes those for which an AMHRII expression score is of 1.5 or more has been determined.

The membranous AMHRII expression score may be based on the immuno-histochemical evaluation of the AMHRII expression by the cancer cells tested, and wherein an individual membranous AMHRII score for a given cancer cell sample (i) is assigned as being 0 if no AMHRII expression is detectable, (ii) is assigned as being 1 if a significant AMHRII expression is detected and (iii) is assigned as being 2 if a high AMHRII expression is detected and (iv) is assigned as being 3 if an over-expression of AMHRII is detected.

Indeed, there is a relationship between (i) the score assigned to the membranous AMHRII expression level through the above-described immuno-histochemical evaluation and (ii) the mean number of AMHRII proteins expressed per cancer cell. As shown in WO2018189379, the membranous AMHRII expression level, allowing assigning an individual membranous AMHRII score, may also be assessed by determining the mean number of membranous AMHRII proteins per cell, starting from a sample of tumor cells that has been previously collected from a patient affected with a cancer according to the invention.

According to the above embodiments of determining responsiveness of an individual affected with a cancer according to the invention to a treatment with an ADC of the invention, a membranous AMHRII expression score is determined, for a given cancer of the invention cell sample, by taking into account both (i) the frequency of AMHRII-expressing cells in the said cancer cell sample and (ii) the level of AMHRII expression by the said AMHRII-expressing cells. Typically, an AMHRII expression score of a given cancer cell sample is determined by the following formula (I):
E-SCORE=FREQ x AMHRII_LEVEL, wherein
- E-SCORE means the AMHRII expression score value for a given cancer cell sample,
- FREQ means the frequency of the cells contained in the said cancer cell sample for which membrane AMHRII expression is detected, and
- AMHRII LEVEL means the level of expression of AMHRII by the AMHRII-expressing cells contained in the said given cancer cell sample.

Illustratively, a E-SCORE of 1.0 is determined for a given cancer according to the invention cell sample wherein (i) 50% of the cells express AMHRII (FREQ value of 0.5) and (ii) the AMHRII expression level (AMHRII_LEVEL) is of 2.

In preferred embodiments, an AMHRII expression score (or E-SCORE) is determined by immunohistological methods as shown in the examples herein. According to these preferred embodiments, AMHRII membrane expression is assessed by using a detectable antibody specific for AMHRII and by (i) determining the frequency of cells having the said anti-AMHRII antibody bound thereto and (ii) determining the intensity of the signal generated by the said detectable anti-AMHRII antibody after its binding to the membrane-expressed AMHRII.

Although, AMHRII-expressing cancer cells having an AMHRII expression score of 1.5 or more have been determined for various cancers, albeit to distinct frequencies. Illustratively, it has been shown that cancer cells derived from colon tumors are classified as AMHRII positive (i.e. having an AMHRII score of 1.5 or more) with a higher frequency than cancer cells derived from head and neck cancer.

For determining the level of AMHRII membrane expression, detection of AMHRII at the cell membrane shall be most preferably performed by using an anti-AMHRII monoclonal antibody having a high affinity and high specificity for AMHRII.

Further, determination of AMHRII expression by an immuno-histochemical method with the view of determining a AMHRII score most preferably involves a careful pretreatment of the tissue sample before contacting the said sample with an appropriate detection reagent (e.g. a high affinity anti-AMHRII monoclonal antibody such as monoclonal 3C23K antibody, having a Kd value of 55.3pM for binding to AMHRII). Sample pretreatment shall allow increasing the availability to the detection reagent of the AMHRII molecules expressed at the cell surface. Illustratively, staining method comprises an appropriate combination of specific steps such as (i) a high-temperature dewaxing by exposure to a microwave source and (ii) a system for amplifying the signal generated by the binding of an AMHRII-binding reagent, such as a biotinylated anti-AMHRII antibody that may be subsequently complexed with a streptavidin-conjugated detectable reagent. A pretreatment dewaxing step has appeared to be important for reversing the detection signal extinction effect due to the prior tissue fixation step. The inventors have shown that AMHRII detectability is particularly sensitive to the action of formalin which is used for the tissue fixation step.

In the context of the present invention, this means that an ADC of the invention will be a useful therapeutic agent with a higher frequency for treating patients affected with a colon cancer than for treating patients affected with a head and neck cancer. This also means that, although an ADC of the invention may be a relevant therapeutic agent for treating patients affected with head and neck cancer, it will be preferred to test previously for the AMHRII expression of the tumor-derived cancer cells for deciding that a specific patient will be administered with an ADC of the invention.

As used herein, the interchangeable terms *"subject"* and *"individual"* denote a mammal, such as a rodent, a feline, a canine, or a primate. Preferably an individual according to the invention is a human being.

As mentioned above, the present invention relates to antibodies and fragments thereof, in particular monoclonal antibodies and fragment thereof, directed against the human Anti-Müllerian Hormone type II receptor (AMHRII), and coupled to a drug. They are thus referred to as an Antibody Drug Conjugate (ADC).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with the invention may be made by the hybridoma method first described by Kohler et al, Nature 256:495 (1975), or may be made by recombinant DNA methods (see, e.g., U.S. Patent No. 4,816,567). The "monoclonal antibodies" may also be isolated from phage antibody libraries using the techniques described in Clackson et al, Nature 352:624-628 (1991) or Marks et al, J. Mol Biol. 222:581-597 (1991), for example.

In the context of the invention, the terms "treating", "treatment" and the like mean reversing, alleviating, slowing the progression of a particular disease, and in particular of an AMHRII expressing cancer, and can even mean completely curing a particular disease, and in particular an AMHRII expressing cancer.

In the context of the present invention, the term "preventing" and the like mean reducing the likelihood, for a given individual, of having a particular disease, and in particular of having an AMHRII expressing cancer.

### Antibody Drug Conjugate (ADC)

An ADC of the present invention if of formula (I):

(D-Lk₁-C(O)-Lk₂-C₂H₄-NH-Lk₃)ₙ-Ab (I)

The Ab part represents an anti-AMHRII antibody, or a fragment thereof, comprising:
(a) a heavy chain wherein the variable domain comprises:
   - a H-CDR1 having a sequence set forth as SEQ ID NO: 1;
   - a H-CDR2 having a sequence set forth as SEQ ID NO: 2; and
   - a H-CDR3 having a sequence set forth as SEQ ID NO: 3;
   and
(b) a light chain wherein the variable domain comprises:
   - a L-CDR1 having a sequence set forth as SEQ ID NO: 4,
   - a L-CDR2 having a sequence set forth as SEQ ID NO: 5; and
   - a L-CDR3 having a sequence set forth as SEQ ID NO: 6.

According to this definition, it means that a fragment of the said anti-AMHRII antibody according to the invention comprises:
(a) a heavy chain wherein the variable domain comprises:
   - a H-CDR1 having a sequence set forth as SEQ ID NO: 1;
   - a H-CDR2 having a sequence set forth as SEQ ID NO: 2; and
   - a H-CDR3 having a sequence set forth as SEQ ID NO: 3;
   and
(b) a light chain wherein the variable domain comprises:
   - a L-CDR1 having a sequence set forth as SEQ ID NO: 4,
   - a L-CDR2 having a sequence set forth as SEQ ID NO: 5; and
   - a L-CDR3 having a sequence set forth as SEQ ID NO: 6.

Antibodies that specifically bind to AMHRII may be produced by any technique known in the art. Thus, antibodies useful for the production of ADC, as described herein, can be murine monoclonal antibodies, human monoclonal antibodies, chimeric antibodies or humanized antibodies.

Where chimeric and humanized antibodies are contemplated, the amino acid sequence of the desired sequence, one skilled in the art can readily produce such antibodies, by standard techniques for production of polypeptides. For instance, they can be synthesized using well-known solid phase method, preferably using a commercially available peptide synthesis apparatus (such as that made by Applied Biosystems, Foster City, Calif.) and following the manufacturer's instructions. Alternatively, such antibodies can be synthesized by recombinant DNA techniques as is well-known in the art.

For example, these fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

In certain embodiments, a method of producing an antibody useful for preparing an ADC of the invention can for example comprise the steps consisting of: (i) culturing a transformed host cell under conditions suitable to allow expression of said antibody or polypeptide; and (ii) recovering the expressed antibody. Antibodies of the invention are suitably separated from the culture medium by conventional immunoglobulin purification procedures such as, for example, protein A-Sepharose, hydroxylapatite chromatography, gel electrophoresis, dialysis, or affinity chromatography.

In a particular embodiment, a human chimeric antibody of the present invention can be produced by obtaining nucleic sequences encoding VL and VH domains of a monoclonal antibody, constructing a human chimeric antibody expression vector by inserting the nucleic sequences encoding VL and VH domains of said monoclonal antibody into an expression vector genes encoding human antibody CH and human antibody CL sequences.

The CH domain of a human chimeric antibody may be any region which belongs to human immunoglobulin, but those of IgG class are suitable and any one of subclasses belonging to IgG class, such as IgG1, IgG2, IgG3 and IgG4 can also be used.

For the CL portion of a human chimeric antibody, it may be a kappa class or lambda class light chain constant region. Methods for producing chimeric antibodies involve conventional recombinant DNA and gene transfection techniques and are well known in the art.

Humanized antibodies may be produced by obtaining nucleic acid sequences encoding CDR domains and constructing a humanized antibody according to techniques known in the art. Methods for producing humanized antibodies based on conventional recombinant DNA and gene transfection techniques are well known in the art. Antibodies can be humanized using a variety of techniques known in the art including, for example, CDR-grafting, veneering or resurfacing; and chain shuffling. The general recombinant DNA technology for preparation of such antibodies is also known.

A monoclonal antibody against Mullerian Hormone type II receptor (and humanized derivatives thereof) has been developed for the treatment of ovarian cancer (see EP 2097453B1 and US Patent No. 8,278,423).

In a particular embodiment, human monoclonal antibodies that specifically bind to AMHRII can be used for the preparation of ADCs according to the invention.

In particular, an antibody, or a fragment thereof, present in an ADC of the invention can be such that the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8.

According to this embodiment, the fragment of an antibody of an ADC of the invention is such that it comprises a heavy chain variable region having the amino acid sequence set forth as SEQ ID NO: 7 and/or a light chain variable region having the amino acid sequence set forth as SEQ ID NO: 8, and in particular comprises the heavy chain variable region of said antibody having the amino acid sequence set forth as SEQ ID NO: 7 and the light chain variable region having the amino acid sequence set forth as SEQ ID NO: 8.

According to another embodiment, an antibody present in an ADC of the invention can be such that the heavy chain of said antibody has the amino acid sequence set forth as SEQ ID NO: 9 and/or the light chain of said antibody has the amino acid sequence set forth as SEQ ID NO: 10, preferably the heavy chain of said antibody has the amino acid sequence set forth as SEQ ID NO: 9 and the light chain of said antibody has the amino acid sequence set forth as SEQ ID NO: 10.

D in formula (I) according to the invention represents a PBD dimer of formula (II): 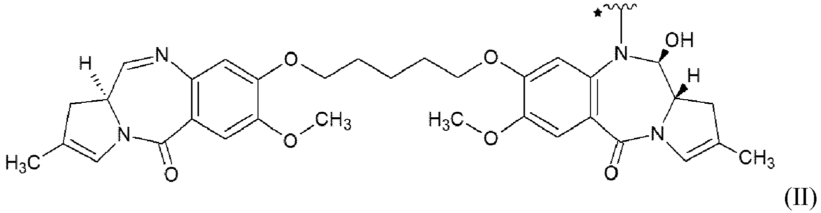 in which ★ represents the link to the LK₁ group of the formula (I).

This payload is a DNA-damaging pyrrolobenzodiazepine (PBD) dimer toxin which has been demonstrated as inducing durable tumor regression *in vivo* across multiple patient-derived xenograft (PDX) tumor models but is also known for its significant toxicity *in vivo* when conjugated to certain antibodies. As illustrated in Rudin et al., Lancet. Oncol. 2017; 18: 42-51, with the DLL3-targeted antibody-drug conjugate (ADC) SC16LD6.5, which is comprised of a humanized anti-DLL3 monoclonal antibody conjugated to this toxin, the administration of this ADC in cynomolgus monkeys in particular led to dose related skin lesions appearing near the end of the first dosing cycle of a schedule of 3 doses administered every three weeks. These were grossly characterized by scattered areas of hyperpigmentation ranging in size from <1 to ∼10 cm. The number of lesions and apparent clinical severity increased with dose (tested doses were 0.5, 1 and 3 mg/kg). all of the animals dosed at 3 mg/kg became less mobile, presumably related to pain, and two animals demonstrated sufficient debility to warrant euthanasia. The remaining 3 mg/kg animals were even deemed too unfit to receive their second dose of ADC. The skin lesions also appeared on animals receiving less than 1 mg/kg of the said ADC. This document states that the toxicity observed with the skin lesions was probably antigen-independent.

However, the inventors unexpectedly identified (data not shown) that an ADC according to the invention, GM104, used in exactly the same conditions on cynomolgus animals and administered at a dose of 1.0 mg/kg or 1.5 mg/kg, does not lead to any visible skin lesion, while the mean DAR of the two different ADC SC16LD6.5 and GM104 is similar (=2).

This payload is linked to the antibody Ab through a linker represented in the formula (I) as -Lk₁-C(O)-Lk₂-C₂H₄-NH-Lk₃-.

Lk₁ represents a cathepsin B-cleavable valine-alanine dipeptide of formula (III): 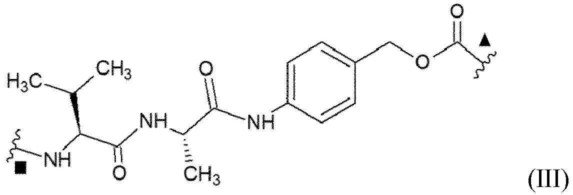 in which ▲ represents the link to the D group and ■ represents the link to the -C(O)- of formula (I).

As mentioned above, Lk₁ is cleavable under physiological intracellular conditions, in particular is cleavable by an intracellular protease, which allows separating the payload from the antibody of an ADC of the invention.

LK₂ represents a group comprising at least one ethylene glycol unit -CH₂-CH₂-O-. According to the present invention, the orientation of the -CH₂-CH₂-O- unit(s) in formula (I) is such that it is read from the (-D-Lk1-C(O)-) side to the (-C2H4-NH-Lk₃-) side.

If more than two ethylene glycol units are present, Lk₂ represents a polyethylene glycol (PEG). This polymer may contain a single linear chain, or may have branched morphology composed of many chains either small or large, in which case it will contain branching groups, typically containing >CH-, as for example in -(O-CH₂-)₂CH-. It may optionally be derivatized or functionalized in any desired way.

It may for example carry an additional therapeutic agent, or a labelling agent.

In a particular embodiment, Lk₂ represents a linear polyethylene glycol.

Accordingly, when Lk2 represents a linear polyethylene glycol, and as explained above regarding the orientation of these units, formula (I) could also be represented as follows:

D-Lk₁-C(O)-(CH₂-CH₂-O)x-C₂H₄-NH-Lk₃)ₙ-Ab

with x represented an integer equal or superior to 1 and in particular from 2 to 14, more particularly from 4 to 12, more particularly from 6 to 10, and even more particularly x can be 8.

In a particular embodiment, Lk₂ represents a group consisting of from 2 to 14 ethylene glycol units, in particular of from 4 to 12 ethylene glycol units and more particularly of from 6 to 10 ethylene glycol units. In particular, Lk₂ can represent a linear group consisting of from 2 to 14 ethylene glycol units, in particular of from 4 to 12 ethylene glycol units and more particularly of from 6 to 10 ethylene glycol units.

More particularly, Lk₂ can represent a group consisting of 8 ethylene glycol units, in particular a group consisting of 8 linear ethylene glycol units.

According to this embodiment, and as mentioned above, this means that the formula (I) can in this case be read as follows:

(D-Lk₁-C(O)-(CH₂-CH₂-O)₈-C₂H₄-NH-Lk₃)ₙ-Ab

According to the present invention, LK₃ in an ADC of the invention represents a linker of formula (IV): 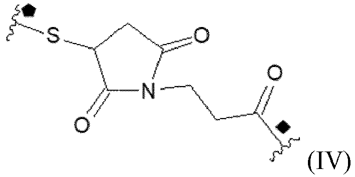 in which ◆ represents the link to the -C₂H₄- of formula (I) and 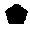 represents the link to the antibody or its fragment thereof.

This maleimide indeed possess an excellent reactivity with the sulfhydryl group present on the Ab antibody or fragment thereof, in particular in the cysteine amino acids of the antibody or fragment thereof.

In the prior art, one negative aspect of utilizing maleimide chemistry for cysteine linkage has been indicated as being its chemical instability in plasma, including a retro-Michael reaction which results in premature loss of the drug-linker from the ADC.

On the contrary, the results present in the present text illustrate that an ADC according to the invention unexpectedly demonstrates an improved half-life compared to an ADC outside the invention.

As mentioned previously, up to 10 (D-Lk₁-C(O)-Lk₂-C₂H₄-NH-Lk₃) groups (n is an integer from 1 to 10 in formula (I)) are linked to the antibody or its fragment thereof in an ADC of the present invention. These different groups are in particular linked to the antibody or its fragment thereof through different cysteines of the antibody or its fragment thereof.

In a particular embodiment, n is an integer from 1 to 8, more particularly from 1 to 7, in particular from 1 to 6, even more particularly from 1 to 5, more particularly from 1 to 4, and in particular from 1 to 3.

In a particular embodiment, n is 1 or 2, i.e. 1 or 2 groups are linked to the antibody or its fragment thereof in an ADC of the present invention. Accordingly, an ADC of the invention can in particular have a Drug-to-Antibody ratio DAR of from 1 to 2. More particularly, 2 groups can be linked to the antibody or its fragment thereof in an ADC of the present invention.

When more than 1 (D-Lk₁-C(O)-Lk₂-C₂H₄-NH-Lk₃) group is present in an ADC of the invention, each (D-Lk₁-C(O)-Lk₂-C₂H₄-NH-Lk₃) group can be identical or different from one another. In a particular embodiment, when more than 1 (D-Lk₁-C(O)-Lk₂-C₂H₄-NH-Lk₃) group is present in an ADC of the invention, all the (D-Lk₁-C(O)-Lk₂-C₂H₄-NH-Lk₃) groups are identical.

In a particular embodiment, an ADC of the invention is such that D, Lk₁ and Lk₃ are as defined in formula (I) and:
- Lk₂ represents a linear group consisting of 8 ethylene glycol units;
- n is 1 or 2, and in particular 2; and
- Ab represents:
   an antibody, or a fragment thereof, wherein the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8; or
   an antibody wherein the heavy chain of said antibody has the amino acid sequence set forth as SEQ ID NO: 9 and/or the light chain of said antibody has the amino acid sequence set forth as SEQ ID NO: 10, and in particular represent an antibody wherein the heavy chain of said antibody has the amino acid sequence set forth as SEQ ID NO: 9 and the light chain of said antibody has the amino acid sequence set forth as SEQ ID NO: 10.

In a particular embodiment of the invention, an ADC according to the invention has the following formula: 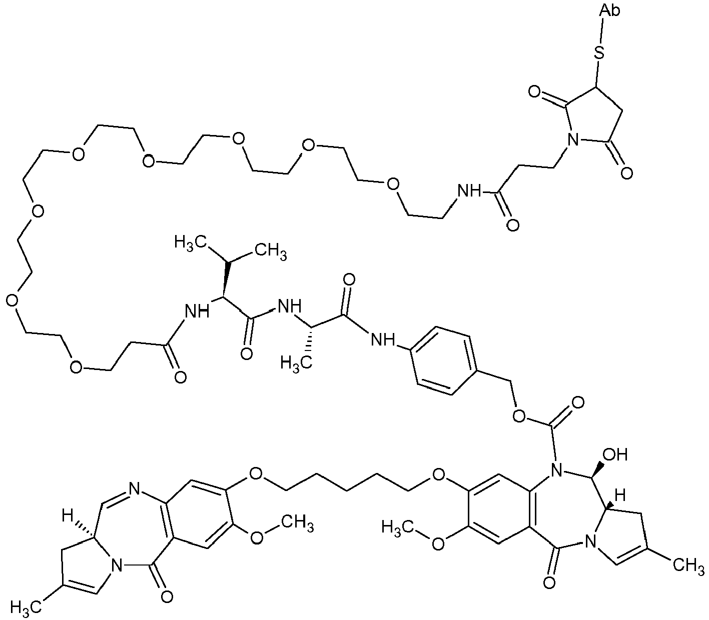 wherein Ab represents an anti-AMHRII antibody, or a fragment thereof, as defined in the present text, i.e. an anti-AMHRII antibody, or a fragment thereof, comprising:
(a) a heavy chain wherein the variable domain comprises:
   - a H-CDR1 having a sequence set forth as SEQ ID NO: 1;
   - a H-CDR2 having a sequence set forth as SEQ ID NO: 2; and
   - a H-CDR3 having a sequence set forth as SEQ ID NO: 3;
   and
(b) a light chain wherein the variable domain comprises:
   - a L-CDR1 having a sequence set forth as SEQ ID NO: 4,
   - a L-CDR2 having a sequence set forth as SEQ ID NO: 5; and
   - a L-CDR3 having a sequence set forth as SEQ ID NO: 6;
in particular, an antibody, or a fragment thereof, wherein the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8;
and more particularly an antibody wherein the heavy chain of said antibody has the amino acid sequence set forth as SEQ ID NO: 9 and/or the light chain of said antibody has the amino acid sequence set forth as SEQ ID NO: 10, preferably the heavy chain of said antibody has the amino acid sequence set forth as SEQ ID NO: 9 and the light chain of said antibody has the amino acid sequence set forth as SEQ ID NO: 10.

An ADC according to the instant application may moreover be labelled with a radioisotope by any method known to the art for use in imaging or therapy. For example imaging radioisotopes, include but are not limited to, I¹²³, I¹²⁴, In¹¹¹, Re¹⁸⁶, Re¹⁸⁸. ADC may be also labelled with a spin label for nuclear magnetic resonance (NMR) imaging (also known as magnetic resonance imaging, MRI). Spin labels for such a use include iodine-123, iodine-131, indium-III, fluorine-19, carbon-13, nitrogen-15, oxygen-17, gadolinium, manganese or iron.

### Pharmaceutical compositions

An ADC of the invention may be combined with a pharmaceutically acceptable medium in order to form a pharmaceutical composition.

Accordingly, another object of the invention is a pharmaceutical composition comprising, in a pharmaceutically acceptable medium, at least one ADC of the invention.

The terms "pharmaceutical" and "pharmaceutically acceptable" refers to molecular entities and compositions that do not produce an adverse, allergic or other untoward reaction when administered to a mammal, especially a human being.

A pharmaceutically acceptable medium refers to a non-toxic solid, semi-solid or liquid filler, diluent, encapsulating material or formulation auxiliary of any type.

A pharmaceutical composition of the invention contains at least one vehicle/medium which can in particular be pharmaceutically acceptable for a formulation capable of being injected. Such medium may in particular be isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. To prepare pharmaceutical compositions, an effective amount of the ADC may be dissolved or dispersed in a pharmaceutically acceptable carrier or aqueous medium.

Pharmaceutical forms suitable for injectable use for example include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

An ADC of the invention can be formulated into a pharmaceutical composition of the invention in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The medium can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid; thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the ADC in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated in the present invention, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated.

The person responsible for administration will, in any event, determine the appropriate dose and schedule of administration for the individual to be treated.

In an embodiment, an ADC of the invention can be administered to an individual as a single weekly ADC dose administered for a period of up to 1 or 2 or 3 months.

In an embodiment, an ADC of the invention can be administered to an individual as consecutive once-weekly ADC doses for up to 4 weeks every 6 months for a period of up to 2 years.

In an embodiment, an ADC of the invention can be administered to an individual as one to 28 daily ADC doses for a period of up to 4 weeks.

In an embodiment, an ADC of the invention can be administered to an individual as two to seven ADC doses, administered once per day, for a period of about two weeks.

Depending on the schedule of administration applied, the quantity of ADC administered with each dose can vary from an individual to another.

### Implementation of the ADC and composition of the invention

Another object of the present invention is an ADC of the invention or a pharmaceutical composition of the invention for its use as a medicament.

In particular, an ADC of the invention, and the compositions of the invention comprising it, may be used for treating any cancer disease associated with the expression of human AMHRII.

Accordingly, a further object of the invention is an ADC of the invention, or a composition of the invention, for its use in the prevention and/or the treatment of an anti-Müllerian hormone type II receptor (AMHRII) expressing cancer in an individual.

The present invention also relates to the use of an ADC of the invention, or a composition of the invention, for the preparation of a medicament for preventing and/or treating an anti-Müllerian hormone type II receptor (AMHRII) expressing cancer in an individual.

The invention moreover relates to the use of an ADC of the invention, or a composition of the invention, for preventing and/or treating an anti-Müllerian hormone type II receptor (AMHRII) expressing cancer in an individual.

The invention furthermore relates to a method for preventing and/or treating an anti-Müllerian hormone type II receptor (AMHRII) expressing cancer in an individual, said method comprising administering to the individual at least on ADC according to the invention, or a composition according to the invention.

According to an embodiment of the invention, the cancer is selected from the group consisting of:
- non-gynecologic cancers, in particular selected from the group consisting of colon cancer; liver cancer; hepatocellular carcinoma; testis cancer; thyroid cancer; gastric cancer; gastrointestinal cancer; bladder cancer; lung cancer, and in particular non-small cell lung cancer; pancreatic cancer; head and neck cancer; kidney cancer; liposarcoma; fibrosarcoma; pleuramesothelioma; melanoma; sarcoma; brain cancer; osteocarcinoma; breast cancer; prostate cancer; leukemia and hematological cancers; and
- gynecologic cancers, in particular selected from the group consisting of ovarian cancer, in particular metastatic ovarian cancer; serous cancer; hypernephroma; endometrioid cancers; colloidal epithelium cancers; germ cell cancers; endometrial cancer; mixed Müllerian malignant tumor of the uterus; leiomyosarcoma; and endometrial stromal sarcoma.

The ADC of the invention is always used in an individual in a therapeutically effective amount.

The terms "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, duration of progression free survival (PFS), the response rates (RR), duration of response, and/or quality of life.

As previously mentioned, the total daily usage of the ADC of the invention and compositions thereof will be decided by the attending physician within the scope of sound medical judgment. The specific therapeutically effective dose level for any particular patient will depend upon a variety of factors including for example the disorder being treated and the severity of the disorder; the specific composition employed; the age, body weight, general health, sex and diet of the patient; the time of administration; the route of administration, and rate of excretion of the specific antibody employed; the duration of the treatment and the drugs optionally used in combination or coincidental with the employed ADC.

The ADC of the invention may indeed be used alone or in combination with any other therapeutic strategy for treating AMHRII expressing cancers, and in particular:
- with radiotherapy treatment or chemotherapeutic treatment; and/or
- with one or more other anti-cancer active agent(s).

An "anticancer agent" is defined as any molecule that can either interfere with the biosynthesis of macromolecules (DNA, RNA, proteins, etc.) or inhibit cellular proliferation, or lead to cell death by apoptosis or cytotoxicity for example. Among the anticancer agents, there may be mentioned alkylating agents, topoisomerase inhibitors and intercalating agents, antimetabolites, cleaving agents, agents interfering with tubulin, monoclonal antibodies.

According to a particular embodiment, the "other" anticancer agent is selected from the group consisting of docetaxel, cisplatin, gemcitabine and a combination of cisplatin and gemcitabine.

According to a particular embodiment, the "other" anticancer agent is selected from the group consisting of paclitaxel or a platinum salt such as oxaliplatin, cisplatin and carboplatin.

The anticancer agent may also be selected from chemotherapeutic agents other than the platinum salts, small molecules, monoclonal antibodies or else anti-angiogenesis peptibodies.

The chemotherapeutic agents other than the platinum salts include the intercalating agents (blocking of DNA replication and transcription), such as the anthracyclines (doxorubicin, pegylated liposomal doxorubicin), the topoisomerase inhibitors (camptothecin and derivatives: Karenitecin, topotecan, irinotecan), or else SJG-136, the inhibitors of histone deacetylase (vorinostat, belinostat, valproic acid), the alkylating agents (bendamustine, glufosfamide, temozolomide), the anti-mitotic plant alkaloids, such as the taxanes (docetaxel, paclitaxel), the vinca alkaloids (vinorelbine), the epothilones (ZK-Epothilone, ixabepilone), the antimetabolites (gemcitabine, elacytarabine, capecitabine), the kinesin spindle protein (KSP) inhibitors (ispinesib), trabectedin or else ombrabulin (combretastatin A-4 derivative).

Among the small molecules there are the poly(ADP-ribose)polymerase (PARP) inhibitors: olaparib, iniparib, veliparib, rucaparib, CEP-9722, MK-4827, BMN-673, the kinase inhibitors, such as the tyrosine kinase inhibitors (TKI) among which there may be mentioned the anti-VEGFR molecules (sorafenib, sunitinib, cediranib, vandetanib, pazopanib, BIBF 1120, semaxanib, Cabozantinib, motesanib), the anti-HER2/EGFR molecules (erlotinib, gefitinib, lapatinib), the anti-PDGFR molecules (imatinib, BIBF 1120), the anti-FGFR molecules (BIBF 1120), the aurora kinase/tyrosine kinase inhibitors (ENMD-2076), the Src/Abl kinase inhibitor (Saracatinib), or also Perifosine, Temsirolimus (mTOR inhibitor), alvocidib (cyclin-dependent kinase inhibitor), Volasertib (inhibitor of PLK1 (polo-like kinase 1) protein, LY2606368 (inhibitor of checkpoint kinase 1 (chk 1), GDC-0449 (Hedgehog Pathway Inhibitor), Zibotentan (antagonist of the ETA-receptor), Bortezomib, Carfilzomib (proteasome inhibitor), cytokines such as IL-12, IL-18, IL-21, INF-alpha, INF-gamma.

Among the antibodies, there may be mentioned, the anti-VEGF: bevacizumab, the anti-VEGFR: ramucirumab, the anti-HER2/EGFRs: trastuzumab, pertuzumab, cetuximab, panitumumab, MGAH22, matuzumab, anti-PDGFR alpha: IMC-3G3, the anti-folate receptor: farletuzumab, the anti-CD27: CDX-1127, the anti-CD56: BB-10901, the anti-CD105: TRC105, the anti-CD276: MGA271, the anti-AGS-8: AGS-8M4, the anti-DRS: TRA-8, the anti-HB-EGF: KHK2866, the anti-mesothelins: amatuximab, BAY 94-9343 (immunotoxin), catumaxomab (EpCAM/CD3 bispecific antibody), the anti-IL2R: daclizumab, the anti-IGF-lR: ganitumab, the anti-CTLA-4: ipilimumab, the anti-PDl: nivolumab and pembrolizumab, the anti-CD47: Weissman B6H12 and Hu5F9, Novimmune 5A3M3, INHIBRX 2A1, Frazier VxP037-01LC1 antibodies, the anti-Lewis Y: Hu3S193, SGN-15 (immunotoxin), the anti-CA125: oregovomab, the anti-HGF: rilotumumab, the anti-IL6: siltuximab, the anti-TR2: tigatuzumab, the anti-alpha5 betal integrin: volociximab, the anti-HB-EGF: KHK2866. The anti-angiogenesis peptibodies are selected from AMG 386 and CVX-241.

The ADC of the invention and the "other anticancer agent" may be combined within one and the same pharmaceutical composition, or may be used in the form of separate pharmaceutical compositions, which may be administered simultaneously or sequentially. In particular, the products may be administered separately, namely either concomitantly, or independently, for example with a time gap.

The present invention is further illustrated by, without in any way being limited to, the examples below.

### EXAMPLES

### Example 1: Preparation of the anti-AMHRII antibodies and antibody-drug conjugate (ADC)

The ADC of the invention used, named GM104, is composed of the antibody 3C23E having the heavy chain of amino acid sequence set forth as SEQ ID NO: 9 and the light chain of amino acid sequence set forth as SEQ ID NO: 10 linked to Tesirine.

The antibody 3C23E is prepared by transient expression in CHO cell lines following WO2011/141653. PBD-based ADC is prepared by conjugation with payload Tesirine as described in WO2014/057074. Conjugation protocol follows WO2015/157595 with mutatis mutandis modifications.

The 3C23E antibodies are first completely reduced overnight with DTT, then excess DTT is removed by TFF, the antibodies are then re-oxidised using dehydroascorbic acid (DHAA) and filtered before conjugation. Conjugation with Tesirine is carried out at room temperature with 5-10 molar equivalent excess to give drug to antibody ratios (DAR) of 1.7-1.8. Excess Tesirine payload is removed by buffer exchange using TFF and the final ADC are formulated into a formulation buffer during the same TFF.

The comparative ADC GM103, derived from the antibody 3C23K, is prepared according to WO2017/025458: 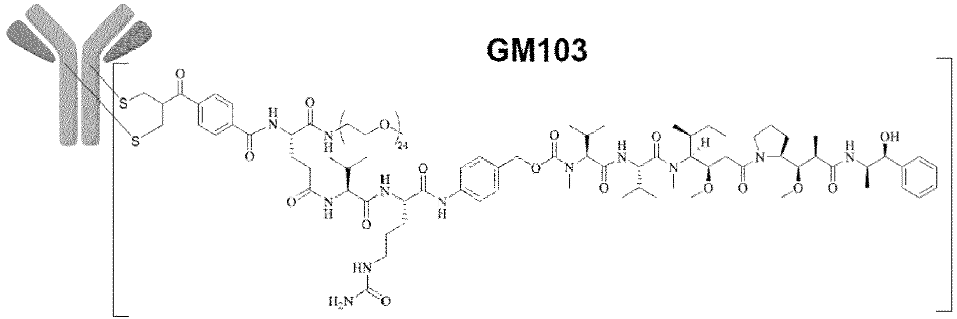 the toxin of GM103 being as described in WO2016/063006.

### Example 2: In vitro cell proliferation and cytotoxic assays

The effects of the prepared ADC on cells viability is measured using resazurin dye reduction assay (CellTiter Blue, Promega).

Cells are plated at 8000 cells per well onto 96-well plate (BD Falcon). After 24 hours at 37°C under 5% CO₂, cells are incubated with serially diluted ADCs in triplicate. Plates are incubated at 37°C under 5% CO₂ for a further 72 hours. CellTiter Blue reagent that enable quantification of viable cells by measurement of metabolic activity is added and plates are read with a fluorometer after a minimum of 2 hours with excitation and emission wavelengths of 544 nm and 590 nm respectively. Anti-proliferative effects are evaluated on COV434 AMHRII transfected cell line (∼80,000 receptors).

Data are analyzed using GraphPad Prism and IC50 values, defined as the concentration of antibodies that results in 50% reduction of viability compared with untreated cells, are determined using a 4-parameter non-linear regression model.

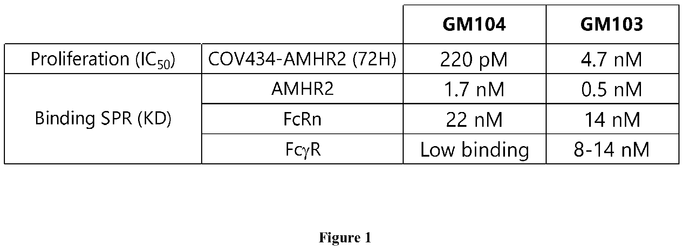

The results obtained on the proliferation of the cells are represented in the Table of Figure 1.

It can be observed that *in vitro,* the IC50 obtained for GM104 ADC according to the invention is higher, and thus is less good, than the IC50 obtained with GM103 ADC, which is outside of the invention and also specifically targets AMHRII.

### Example 3: In vitro measure of the binding kinetic parameters of the ADCs

Immobilization of AMHRII is performed on a Bia3000 apparatus at 25°C in HBS-EP (HEPES 10mM, NaCl 150mM, EDTA 3mM, P20 surfactant 0.005%) at 10µl/min flow rate on CM5 sensor chip using EDC/NHS (1-Ethyl-3-3-dimethylaminopropyl carbodiimidehydrochloride) activation according to the manufacturer's instructions (GEHealthcare).

Briefly, carboxyl functions of CM5 sensor chip are activated with 0.4M EDC/ 0.1M NHS during 7min.

A solution of AMHRII at 0.8-1.25µg/ml in sodium acetate buffer pH 4.5 is injected, and the injection stopped at a level around 600-700RU. An injection of 1M ethanolamine hydrochloride pH8.5 is used during 7 min to neutralize the activation. AMHRII is covalently immobilized at 600-700 RU level on Flowcell. A control reference surface is prepared using the same chemical treatment of the Flowcell 1 without injection of AMHRII.

All kinetic measurements are performed on a Bia3000 apparatus at 25°C in PBS pH 7.4 containing 0.005% of P20 surfactant at 50µl/min on control and AMHRII Flowcells. The antibody is injected during 180s at concentrations given in the appendix. After a dissociation step of 400s in running buffer, sensor surfaces are regenerated using one pulse of 15 µl of Gly-HCL pH1.7. All the sensorgrams are corrected by subtracting the low signal from the control reference surface and blank buffer. Kinetic parameters are evaluated from the sensorgrams using a Langmuir 1:1 model on the BiaEvaluation software 4.2.
In a preliminary experiment, the binding of all the mAb and ADC at one single concentration of 20nM are compared on AMHRII-coated surface at 700 RU.

For kinetic studies all the mAb and ADC are injected at various concentrations (0.7nM-100nM). The sensorgrams are evaluated by L1:1 fitting model (BiaEvaluation 4.2 software) starting of minimum 3 (n=3) separated experiments or fits to obtain average values for ka, kd and KD with standard deviation (SD). Several experiments are performed on the same AMHRII-coated surface after verification of the responses stability to 10nM injection of 3C23K before and after the experiment series with each mAb/ADC. All results are obtained from two AMHRII-coated Flowcells.

The results obtained are represented in the Table of Figure 1.

It can be observed that *in vitro,* the binding to AMHRII obtained for GM104 ADC according to the invention is higher, and thus is less good, than the binding obtained with GM103 ADC, which is outside of the invention.

### Example 4: Binding of the anti-AMHRII ADC to the FCγRIIIA receptor (CD16A)

Immobilization of anti-his is performed on a T200 apparatus at 25°C in PBS containing 0.05% P20 surfactant (PBS-P+) at 10µl/min flow rate on CM5-S sensor chip using EDC/NHS activation according to the manufacturer's instructions (GEHealthcare).

Briefly, carboxyl functions of CM5 sensor chip are activated with a mix of 0.4M EDC/ 0.1M NHS during 7min. A solution of anti-His at 5 µg/ml in sodium acetate buffer pH 4.5 is injected during 7 min. An injection of 1M ethanolamine hydrochloride pH8.5 is used during 7 min to neutralize the activation. Anti-His is covalently immobilized at 8000-10000 RU level on Flowcell 2,3,4. A control reference surface is prepared using the same chemical treatment of the Flowcell 1 without injection of anti-His.

All kinetic measurements are performed on a T200 apparatus at 25°C in PBS-P+ at 100µl/min on the four flowcells. The gamma Fc receptors (FcγR) are captured on immobilized anti-his at 100-200RU during 60s. 5 increasing concentrations of mAb/ADC are injected (injection time =120s). After a dissociation step of 600s in running buffer, sensor surfaces are regenerated using 10µl of Glycine-HCl pH1.7. All the sensorgrams are corrected by subtracting the low signal from the control reference surface and buffer blank injections instead of mAb/ADC.

Kinetic parameters are evaluated from the sensorgrams using a Heterogeneous Ligand or Two-State conformation change or Steady-State on the T200 evaluation software (ref. Karlsson R. method «New insights into BiacoreTM applications» 2012).

The results obtained are represented in the Table of Figure 1.

### Example 5: Assessment of the internalisation of the ADC

Antibody mediated internalization are assessed by flow cytometry analysis. Flow cytometry experiments are done on the SKOV3-AMHRII transfected cell line with high level of AMHRII expression.

Briefly, 5 x 10⁵ cells are incubated with PBD-ADC or the corresponding parental unconjugated antibody at 10µg/ml and incubated 30 min at 4°C. After washes, DMEM/GlutaMax (Gibco) medium, cold or pre-warmed at 37°C, is added and incubated at 4°C or 37°C at different time points (0min, 30min and 60min). Phycoerythrin-conjugated anti-human IgG antibody (1:200, Beckman-Coulter) is added for 30min at 4°C. FACS analysis of the resuspended cells is done on a BD Accuri™ C6 flow cytometer (BD Bioscience).

Internalization of the PBD-ADC antibody occurs in SKOV3-AMHRII high expressing cell line with the same kinetic than parental unconjugated antibodies. No major difference of internalization between the clones is observed. No significant difference is observed between the times of internalization of GM103 and GM104 (Data not shown).

### Example 6: Dose response of ADCs in COV434 AMHRII transfected cell line tumor xenograft in Nude mice

A COV434 AMHRII transfected cell line is cultured as previously described and implanted in female athymic nude mice (Hsd:Athymic Nude-Foxlnu, Harlan Laboratories). 10x10⁶ cells (100µl) are injected in the interscapular mammary fat pad of the mice with matrigel. Mice are allocated to different group according to their tumor volume to give homogenous mean and median tumor volume in each treatment arms.

For each group, 8 mice with established growing tumors and tumors volume ranging from 60 to 200 mm³ are included in the study. GM104 ADC is administered once intravenously at 0.1 mg/kg, 0.3 mg/kg and 1.0 mg/kg while GM103 ADC is administered once intravenously at 5mg/kg.

The corresponding vehicle (PBS) is administrated on the same day. During the whole experimental period, from grafting to study termination, animals are observed every day, for physical appearance, behavior and clinical changes. Tumor volume is evaluated by measuring tumor diameters three times a week and their size was calculated as mm³ = 0.5 x (tumor length, mm) x (tumor width, mm)² with the length and the width are the longest and the shortest diameters respectively.

The results obtained are represented in Figures 2, 3A, 3B et 3C, as well as in the following Table.

The effect of GM104 *in vivo* on the prevention of the tumor growth, and even more particularly on its regression, appears as being clearly superior to the one of GM103, even though GM104 is used in a concentration at least five times inferior to the concentration used for GM103.

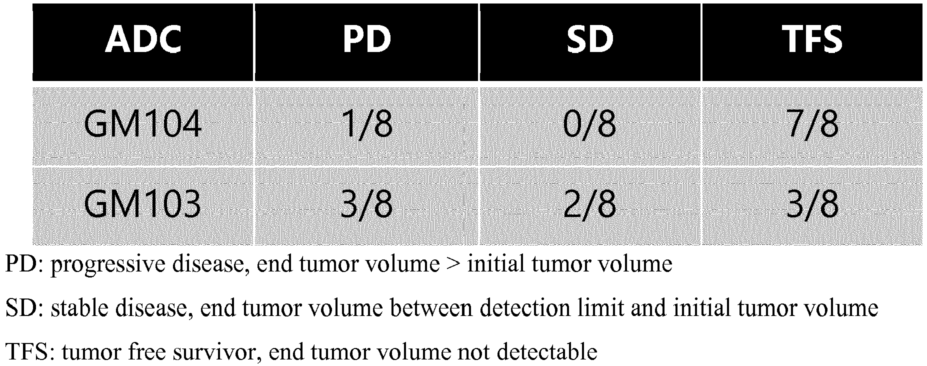

The long-term effect, *in vivo,* of an ADC of the invention appears as being clearly superior to the same effect observed with an ADC not from the invention (GM103). Almost all the individuals from the group treated with an ADC of the invention are indeed completely healed from the cancer.

### Example 7: In vivo efficacy of an ADC according to the invention on LU7298

LU7298, a patient derived xenograft (PDX) from human non-small cell lung cancer patient (NSCLC) is implanted in female NMRI nude mice (NMRI:nu/nu female, Janvier).

Tumor fragments of LU7298 tumors are subcutaneously transplanted in the left flank of nude mice at day 0. At palpable tumor size mice are stratified into groups of 8 mice each (TVmean: 107 mm3, day 23).

GM104 is administered intravenously three times at 1.0 mg/kg (q4dx3) while GM103 is administered once intravenously at 10mg/kg.

Body weight and tumor volume is measured 2x/week. All mice are individually sacrificed at tumor volume >1,5 cm3 (used as survival parameter) or at final day 111, and an autopsy is done. The corresponding vehicle (PBS) is administrated on the same day.

Tumor volume is evaluated by measuring tumor diameters three times a week and their size is calculated as mm³ = 0.5 x (tumor length, mm) x (tumor width, mm)² with the length and the width are the longest and the shortest diameters respectively.

The results obtained are represented in Figures 4A and 4B.

It is firstly observed that the tumors of the individuals treated with GM104 grow slower than the tumors of the individuals from the other groups.

Moreover, almost all the individual of the group treated with GM104 survive for at least the 111 days following the treatment, while on the contrary, more than 50%, or even more than 75%, of the individuals of the other groups die before the 111^{th} day post treatment.

### Example 8: In vivo half-life measures of GM103 and GM104

Pharmacokinetics are performed in male NMRI nude mice (NMRI:nu/nu male, Janvier), with 6 animals per groups. Animals are split in two sub-groups of 3 mice for monitoring at (5 min and 72 hours) and (24h and 240h). Serum concentration of ADC is monitored using an ELISA method using an antibody targeted at the protein portion of the ADC. All test items are injected intravenously as a single injection at 1.0 mg/kg.

Pharmacokinetic evaluation of serum data is performed using WinNonlin 8.0 (Pharsight Corporation, Mountain View, CA, USA) and a non-compartmental model is employed.

The results obtained are represented in Figure 5.

It is observed that unexpectedly the half-life of GM104 is significantly higher than the one of GM103.

| **Sequence number** | **Sequence** |
|---|---|
| SEQ ID NO: 1 | KASGYTFTSYHIH |
| SEQ ID NO: 2 | WIYPGDDSTKYSQKFQG |
| SEQ ID NO: 3 | GDRFAY |
| SEQ ID NO: 4 | RASSSVRYIA |
| SEQ ID NO: 5 | PTSSLES |
| SEQ ID NO: 6 | LQWSSYPWTF |
| SEQ ID NO: 7 | QVRLVQSGAEVKKPGASVKVSCKASGYTFTSYHIHWVR QAPGQRLEWMGWIYPGDDSTKYSQKFQGRVTITRDT SASTAYMELSSLRSEDTAVYYCTRGDRFAYWGQGTLVT VSS |
| SEQ ID NO: 8 | DIQMTQSPSTLSASVGDRVTITCRASSSVRYIAWYQQK PGKAPKLLTYPTSSLESGVPSRFSGSGSGTEFTLTISSLQP DDFATYYCLQWSSYPWTFGGGTKVEIK |
| SEQ ID NO: 9 | QVRLVQSGAEVKKPGASVKVSCKASGYTFTSYHIHWVR QAPGQRLEWMGWIYPGDDSTKYSQKFQGRVTITRDT SASTAYMELSSLRSEDTAVYYCTRGDRFAYWGQGTLVT VSSASTKGPSVFPLAPSSKSTSGGTAALGCLVKDYFPEP VTVSWNSGALTSGVHTFPAVLQSSGLYSLSSVVTVPSSS LGTQTYICNVNHKPSNTKVDKRVEPKSCDKTHTCPPCP APELLGGPSCVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQP REPQVYTLPPSREEMTKNQVSLTCLVKGFYPSDIAVEW ESNGQPENNYKTTPPVLDSDGSFFLYSKLTVDKSRWQ QGNVFSCSVMHEALHNHYTQKSLSLSPGK |
| SEQ ID NO: 10 | DIQMTQSPSTLSASVGDRVTITCRASSSVRYIAWYQQK PGKAPKLLTYPTSSLESGVPSRFSGSGSGTEFTLTISSLQP DDFATYYCLQWSSYPWTFGGGTKVEIKRTVAAPSVFIF PPSDEQLKSGTASVVCLLNNFYPREAKVQWKVDNALQ SGNSQESVTEQDSKDSTYSLSSTLTLSKADYEKHKVYAC EVTHQGLSSPVTKSFNRGEC |
| SEQ ID NO: 11 | PPNRRTCVFFEAPGVRGSTKTLGELLDTGTELPRAIRCLY SRCCFGIWNLTQDRAQVEMQGCRDSDEPGCESLHCD PSPRAHPSPGSTLFTCSCGTDFCNANYSHLPPPGSPGTP GSQGPQAAPGESIWMALVLLGLFLLLLLLLGSIILALLQR KNYRVRGEPVPEPRPDSGRDWSVELQELPELCFSQVIR EGGHAVVWAGQLQGKLVAIKAFPPRSVAQFQAERALY ELPGLQHDHIVRFITASRGGPGRLLSGPLLVLELHPKGSL CHYLTQYTSDWGSSLRMALSLAQGLAFLHEERWQNG QYKPGIAHRDLSSQNVLIREDGSCAIGDLGLALVLPGLT QPPAWTPTQPQGPAAIMEAGTQRYMAPELLDKTLDL QDWGMALRRADIYSLALLLWEILSRCPDLRPDSSPPPF QLAYEAELGNTPTSDELWALAVQERRRPYIPSTWRCFA TDPDGLRELLEDCWDADPEARLTAECVQQRLAALAHP QESHPFPESCPRGCPPLCPEDCTSIPAPTILPCRPQRSAC HFSVQQGPCSRNPQPACTLSPV |
| SEQ ID NO: 12 | MLGSLGLWALLPTAVEA |

## Claims

1. An antibody drug conjugate (ADC) of general formula (I):
(D-Lk₁-C(O)-Lk₂-C₂H₄-NH-Lk₃)ₙ-Ab (I)
wherein:
- D represents a PBD dimer of formula (II): 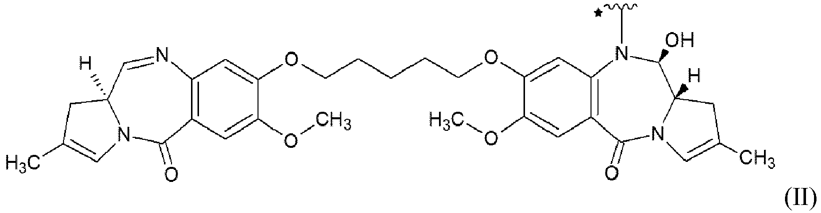 in which ★ represents the link to the Lk₁ group;
- Lk₁ represents a cathepsin B-cleavable valine-alanine dipeptide of formula (III): 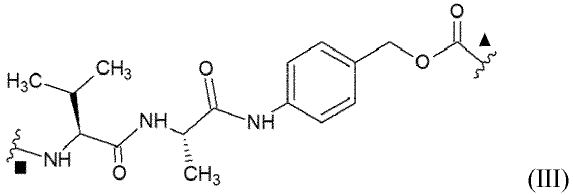 in which ▲ represents the link to the D group and ■ represents the link to the -C(O)-;
- Lk₂ represents a group comprising at least one ethylene glycol unit -CH₂-CH₂-O-;
- Lk₃ represents a linker of formula (IV): 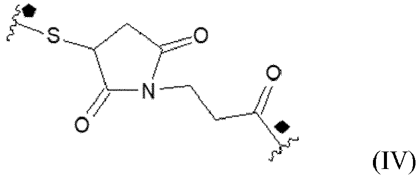 in which ◆ represents the link to the -C₂H₄- group and 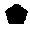 represents the link to the antibody or its fragment thereof;
- n is an integer from 1 to 10; and
- Ab represents an anti-AMHRII antibody, or a fragment thereof, comprising:
(a) a heavy chain wherein the variable domain comprises:
- a H-CDR1 having a sequence set forth as SEQ ID NO: 1;
- a H-CDR2 having a sequence set forth as SEQ ID NO: 2; and
- a H-CDR3 having a sequence set forth as SEQ ID NO: 3;
and
(b) a light chain wherein the variable domain comprises:
- a L-CDR1 having a sequence set forth as SEQ ID NO: 4,
- a L-CDR2 having a sequence set forth as SEQ ID NO: 5; and
- a L-CDR3 having a sequence set forth as SEQ ID NO: 6.

2. The ADC according to claim 1, in which Lk₂ represents a group consisting of from 2 to 14 ethylene glycol units, in particular of from 4 to 12 ethylene glycol units, more particularly of from 6 to 10 ethylene glycol units, and even more particularly of 8 ethylene glycol units.

3. The ADC according to claim 1 or 2, in which Lk₂ represents a linear group consisting of from 2 to 14 ethylene glycol units, in particular a linear group consisting of from 4 to 12 ethylene glycol units, more particularly a linear group consisting of from 6 to 10 ethylene glycol units, and even more particularly a linear group consisting of 8 ethylene glycol units.

4. The ADC according to any one of claims 1 to 3, in which n is an integer from 1 to 8, in particular from 1 to 6, more particularly from 1 to 4, and is even more particularly 1 or 2, in particular 2.

5. The ADC according to any one of claims 1 to 4, in which Ab represents an antibody, or a fragment thereof, wherein the heavy chain variable region of the antibody has the amino acid sequence set forth as SEQ ID NO: 7 and/or the light chain variable region has the amino acid sequence set forth as SEQ ID NO: 8, in particular the heavy chain variable region of said antibody has the amino acid sequence set forth as SEQ ID NO: 7 and the light chain variable region has the amino acid sequence set forth as SEQ ID NO: 8.

6. The ADC according to any one of claims 1 to 5, in which Ab represents an antibody wherein the heavy chain of said antibody has the amino acid sequence set forth as SEQ ID NO: 9 and/or the light chain of said antibody has the amino acid sequence set forth as SEQ ID NO: 10, in particular the heavy chain of said antibody has the amino acid sequence set forth as SEQ ID NO: 9 and the light chain of said antibody has the amino acid sequence set forth as SEQ ID NO: 10.

7. The ADC according to any one of claims 1 to 6, in which D, Lk₁ and Lk₃ are as defined in claim 1 and:
- Lk₂ represents a linear group consisting of 8 ethylene glycol units;
- n is 1 or 2, and in particular 2; and
- Ab represents an antibody as defined in claim 5 or 6.

8. A pharmaceutical composition comprising, in a pharmaceutically acceptable medium, at least one ADC as defined in any one of claims 1 to 7.

9. An antibody drug conjugate (ADC) as defined in any one of claims 1 to 7 or a composition as defined in claim 8, for its use as a medicament.

10. An antibody drug conjugate (ADC) as defined in any one of claims 1 to 7 or a composition as defined in claim 8, for its use in the prevention and/or the treatment of an anti-Müllerian hormone type II receptor (AMHRII) expressing cancer in an individual.

11. The ADC for its use, or composition for its use, according to claim 10, wherein the cancer is selected from the group consisting of:
- non-gynecologic cancers, in particular selected from the group consisting of colon cancer; liver cancer; hepatocellular carcinoma; testis cancer; thyroid cancer; gastric cancer; gastrointestinal cancer; bladder cancer; lung cancer, in particular non-small cell lung cancer (NSCLC); pancreatic cancer; head and neck cancer; kidney cancer; liposarcoma; fibrosarcoma; pleuramesothelioma; melanoma; sarcoma; brain cancer; osteocarcinoma; breast cancer; prostate cancer; leukemia and hematological cancers; and
- gynecologic cancers, in particular selected from the group consisting of ovarian cancer, in particular metastatic ovarian cancer; serous cancer; hypernephroma; endometrioid cancers; colloidal epithelium cancers; germ cell cancers; endometrial cancer; mixed Müllerian malignant tumor of the uterus; leiomyosarcoma and endometrial stromal sarcoma.

12. The ADC for its use, or composition for its use, according to claim 10 or 11, wherein the ADC for its use, or the composition for its use, is administered to the said individual intravenously or at the cancer localisation.

13. The ADC for its use, or composition for its use, according to any one of claims 10 to 12, wherein the individual is a mammal, and in particular a human being.
